# EUROPEAN PATENT SPECIFICATION

(11) **EP 3 010 637 B1**
(45) Date of publication and mention of the grant of the patent: **03.04.2019**
(21) Application number: 14812933.1
(22) Date of filing: 17.06.2014
(51) Int. Cl.: B01J 20/08, B01D 53/58

(54) **A PROCESS FOR THE REMOVAL OF METAL CONTAMINANTS FROM FLUIDS**
VERFAHREN ZUR ENTFERNUNG VON METALLISCHEN VERUNREINIGUNGEN AUS FLÜSSIGKEITEN
PROCÉDÉ D'ÉLIMINATION DE CONTAMINANTS MÉTALLIQUES CONTENUS DANS DES FLUIDES

(30) Priority: 17.06.2013 IN 2038MU2013
(43) Date of publication of application: 27.04.2016
(73) Proprietor: Reliance Industries Limited, Mumbai 400 021 Maharashtra (IN)
(72) Inventor: KUMAR, Prakash, Navi Mumbai 400701 Maharashtra (IN); PURANIK, Vijayalakshmi Ravi, Navi Mumbai 400701 Maharashtra (IN); PATIL, Mallikarjun, Navi Mumbai 400701 Maharashtra (IN); GOPALKRISHNAN, Kalpana, Navi Mumbai 400701 Maharashtra (IN); JASRA, Raksh Vir, Navi Mumbai 400701 Maharashtra (IN)
(74) Representative: Goodman, Simon John Nye
(86) International application number: PCT/IN2014/000403
(87) International publication number: WO 2014/203276

(56) References cited:
- EP-A1- 0 379 394
- WO-A1-95/33680
- US-A- 3 411 879
- US-A1- 2002 014 439
- US-A1- 2005 028 672
- US-A1- 2008 257 150
- US-A1- 2011 079 145

## Description

### FIELD OF THE DISCLOSURE

The present disclosure relates to a process for the removal of metal contaminants from fluids.

### BACKGROUND

Chlorinated polymers find extensive applications in roofing membranes, geomembranes, coated fabrics, cable insulation and as impact modifiers and base polymers. Their excellent physical and mechanical properties such as resistance to chemicals and oils, flame retardancy, tensile strength and resistance to abrasion have resulted in their enormous industrial popularity and applicability.

Chlorinated polymers are usually prepared by reacting chlorine free polymers and/ or copolymers with chlorinating agents, at pre-determined temperature and pressure conditions. Ethylene and hydrochloric acid are the commonly used raw materials in the polymerization processes. However, due to the highly corrosive nature of the above-mentioned chemicals, more specifically in the presence of water, numerous incidences of corrosion of the reaction vessels and pipelines are observed. Furthermore, the corroded surfaces give off metallic particulate matter that enters the polymerization reaction via the raw materials. The presence of metal contaminants in the reaction leads to a pressure drop in the system along with the generation of hot spots in the downstream catalyst bed, leading to reduction in the life of the catalyst. Even further, fouling of the catalyst occurs, which creates unwanted products in the reaction, leading to a decrease in the overall yield.

Different methods have been developed to eliminate the metal contaminants from the raw materials. EP 0618170 suggests a strong basic anion exchange resin for removing iron impurities from hydrochloric acid. EP 0725759 suggests filtration and ion exchange techniques for removing coloring material such as iron and halogen from hydrochloric acid. The method recited in EP 0725759 further includes a step of addition of a reducing agent in the hydrochloric acid, post the removal of the coloring matter. US 3411879 suggests a process for the removal of fluoride ions from aqueous hydrochloric acid by treating it with a treating agent such as activated alumina or silica gel. US 5330735 suggests a process for treating contaminated hydrochloric acid to remove silicone containing materials that may act as impurities such as silane, silanol and siloxane. The process recited in US 5330735 uses a hydrophobic polystyrenic resin. Although there has been significant development in the techniques for the removal of metal contaminants from raw materials, the conventional methods are still accompanied by certain disadvantages such as the inability to remove metal contaminants below certain sizes and the use of expensive reagents and substrates. US 8298311 and EP 0630677 suggest ceramic articles for the purposes of filtration and removal of impurities such as dust. However, the afore-mentioned prior art methods remain silent on use of ceramic materials for removing metal contaminants.

US2008/257150 describes the removal of inorganic compounds from HCl gas using γ-alumina having a BET specific surface area of 10 to 1000m²/g. The inorganic compounds can be in the form of oxides.

The inventors of the present disclosure have envisaged a simple and economic process for the removal of metal contaminants from the raw materials of the polymerization process.

### OBJECTS

Some of the objects of the present disclosure which at least one embodiment herein satisfies are as follows:
It is an object of the present disclosure to provide a process for the removal of metal contaminants from a fluid stream.

It is another object of the present disclosure to provide a simple and cost effective process for the removal of metal contaminants from a fluid stream.

It is yet another object of the present disclosure to provide an environment friendly process for the removal of metal contaminants from a fluid stream.

It is even further an object of the present disclosure to ameliorate one or more problems of the prior art or to at least provide a useful alternative.

Other objects and advantages of the present disclosure will be more apparent from the following description which is not intended to limit the scope of the present disclosure.

### SUMMARY

The present disclosure provides a process for removing metal contaminants from a contaminated fluid stream, comprising the step of contacting said fluid stream with macroporous alpha alumina to obtain a fluid stream containing less than 50 ppm of metal, as defined in claim 1.

The fluid stream is a stream of mineral acid(s). The metal contaminant can be at least one of elemental metals and metal oxides.

The process further comprises a step of obtaining spent macroporous alpha alumina and regenerating active macroporous alpha alumina therefrom by washing said spent macroporous alpha alumina with at least one purified stream selected from the group consisting of a stream of mineral acid(s) and a stream of alkylene(s).

The mineral acid can be at least one corrosive acid selected from the group consisting of hydrochloric acid, hydrofluoric acid, hydrobromic acid, nitric acid, phosphoric acid, sulfuric acid, boric acid and perchloric acid.

In one embodiment the fluid stream is a hydrochloric acid stream.

The metal contaminant can be at least one metal from the group consisting of iron, nickel, copper, chromium, lead, zinc, manganese and oxides thereof.

In one embodiment the metal contaminant is iron oxide.

The step of contacting can be carried out at a temperature ranging between 20 °C and 200 °C.

The step of contacting can be carried out at a pressure ranging between 1 kg/ cm² and 10 kg/ cm².

The step of contacting can be carried out at a gas hourly space velocity (GHSV) ranging between 10,000 per hour and 20,000 per hour.

The shape of said macroporous alpha alumina can be selected from the group of shapes consisting of bead shape and disc shape.

### DETAILED DESCRIPTION

The present disclosure provides a process for obtaining a fluid stream, substantially free of metal contaminants, that is capable of being used as a raw material in the process of preparation of chlorinated polymers. The process of polymerization becomes more cost effective, environment friendly and high yielding by using the purified raw material streams provided by the process of the present disclosure. The fluid stream, substantially free of metal contaminants, is characterized in that the metal content of the stream is less than 50 ppm.

The process of obtaining a fluid stream substantially free of metal contaminants comprises an initial step of providing a fluid stream contaminated with at least one metal contaminant. The fluid stream can be selected from the group consisting of a stream of mineral acid(s) and a stream of alkylene(s). The mineral acid used in the present disclosure is at least one corrosive acid selected from the group that includes but is not limited to hydrochloric acid, hydrofluoric acid, hydrobromic acid, nitric acid, phosphoric acid, sulfuric acid, boric acid and perchloric acid. The metal contaminant contained in the fluid stream includes but is not limited to elemental metal(s) and metal oxide(s). The metal contaminant, in the present disclosure, can be selected from the group consisting of iron, nickel, copper, chromium, lead, zinc, manganese and oxides thereof.

The next step involves contacting the above-mentioned fluid stream with macroporous alpha alumina in order to trap the afore-stated metal contaminants. Macroporous alpha alumina typically withstands a highly acidic and corrosive environment for prolonged periods. Therefore, its use as a molecular sieve is highly effective in the process of the present disclosure. In one embodiment, macroporous alpha alumina with characteristic morphology and integrity is obtained when the precursor gamma alumina is subjected to heat treatment at a temperature ranging between 1500 °C and 1700 °C. The shape of macroporous alpha alumina is selected from the group of shapes consisting of bead shape and disc shape. The step of contacting is carried out at a temperature ranging between 20 °C and 200 °C and at a pressure ranging between 1 kg/ cm² and 10 kg/ cm². The gas hourly space velocity (GHSV) of the fluid stream ranges between 10,000 per hour and 20,000 per hour.

The process of the present disclosure removes the metal contaminants by shape selectivity. This feature is characteristic to the process of the present disclosure. The step of contacting provides a fluid stream free of metal contaminants and spent macroporous alpha alumina. The spent macroporous alpha alumina of the present disclosure may further be regenerated by washing with at least one purified stream selected from the group consisting of a stream of mineral acid(s) and a stream of alkylene(s). The mineral acid of the present disclosure is at least one corrosive acid that includes but is not limited to hydrochloric acid, hydrofluoric acid, hydrobromic acid, nitric acid, phosphoric acid, sulfuric acid, boric acid and perchloric acid.

In one embodiment of the present disclosure, hydrochloric acid vapors, substantially free of iron oxide contaminants, are obtained by passing the dry hydrochloric acid vapors containing iron oxide contaminants, through a fixed bed containing macroporous alpha alumina.

The present disclosure will now be explained with the help of the following non-limiting examples:

### Example 1: Removing metal contamination from a HCl stream

300 g of porous alpha alumina was charged in an adsorbent column trap comprising SS316 and was activated at 200°C for 4 hours under air flow. A dry hydrochloric acid stream was then passed through the column at 160 °C, at a flow rate of 1 liter per minute for 24 hours. The outlet stream emerging from the bed was analyzed for Fe content at the end of 24 hours. Presence of Fe in the outlet stream was analyzed by scrubbing the stream in deionized water. After scrubbing, the deionized water turned light yellow in color. The outlet stream-scrubbed deionized water was then subjected to Inductively Coupled Plasma Optical Emission spectrometer (ICP-OES) analysis which showed 38 ppm of Fe present in the free HCl.

Determination of the Fe content: The dry HC1, at 160 °C, obtained from a Vinyl Chloride Monomer (VCM) plant was analyzed for Fe contamination. The out-coming hydrochloric acid stream was analyzed by scrubbing the stream in deionized water for about 15 minutes. The scrubber was made by filling 50 ml of double distilled water in a glass trap. After scrubbing, the deionized water turned light yellow in color and was further subjected to ICP-OES analysis. The ICP analysis showed 38 ppm of iron present in the outlet stream as compared to the 600 ppm of iron present in a stream not subjected to porous alpha alumina adsorption.

The embodiments herein and the various features and advantageous details thereof are explained with reference to the non-limiting embodiments in the description. Descriptions of well-known components and processing techniques are omitted so as to not unnecessarily obscure the embodiments herein. The examples used herein are intended merely to facilitate an understanding of ways in which the embodiments herein may be practiced and to further enable those of skill in the art to practice the embodiments herein. Accordingly, the examples should not be construed as limiting the scope of the embodiments herein.

The foregoing description of the specific embodiments will so fully reveal the general nature of the embodiments herein that others can, by applying current knowledge, readily modify and/or adapt for various applications such specific embodiments without departing from the generic concept, and, therefore, such adaptations and modifications should and are intended to be comprehended within the meaning and range of equivalents of the disclosed embodiments. It is to be understood that the phraseology or terminology employed herein is for the purpose of description and not of limitation. Therefore, while the embodiments herein have been described in terms of preferred embodiments, those skilled in the art will recognize that the embodiments herein can be practiced with modification within the spirit and scope of the embodiments as described herein.

### TECHNICAL ADVANTAGES

The present disclosure that relates to a process for the removal of metal contaminants from a fluid stream has the following technical advantages:
The process of the present disclosure eliminates the phenomenon of catalyst poisoning during the manufacture of chlorinated polymers.

Further, the process of the present disclosure is capable of removing iron oxide contaminants of small sizes that cannot be removed by the known methods.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

The use of the expression "at least" or "at least one" suggests the use of one or more elements or ingredients or quantities, as the use may be in the embodiment of the invention to achieve one or more of the desired objects or results.

The numerical values given for various physical parameters, dimensions and quantities are only approximate values and it is envisaged that the values higher than the numerical value assigned to the physical parameters, dimensions and quantities fall within the scope of the invention and the claims unless there is a statement in the specification to the contrary.

## Claims

1. A process for removing metal contaminants from a contaminated fluid stream comprising the step of contacting said fluid stream with macroporous alpha alumina to obtain a fluid stream containing less than 50 ppm of metal, wherein said fluid stream is a stream of mineral acid(s).

2. The process as claimed in claim 1, wherein said metal contaminant is at least one of elemental metals and metal oxides.

3. The process as claimed in claim 1 further comprising a step of obtaining spent macroporous alpha alumina and regenerating active macroporous alpha alumina therefrom by washing said spent macroporous alpha alumina with at least one purified stream of mineral acid(s), wherein said mineral acid is at least one corrosive acid selected from the group consisting of hydrochloric acid, hydrofluoric acid, hydrobromic acid, nitric acid, phosphoric acid, sulfuric acid, boric acid and perchloric acid.

4. The process as claimed in claim 1, wherein said fluid stream is a hydrochloric acid stream.

5. The process as claimed in claim 1, wherein said metal contaminant is at least metal from the group consisting of iron, nickel, copper, chromium, lead, zinc, manganese and oxides thereof.

6. The process as claimed in claim 1, wherein said metal contaminant is iron oxide.

7. The process as claimed in claim 1, wherein said step of contacting is carried out at a temperature ranging between 20 °C and 200 °C.

8. The process as claimed in claim 1, wherein said step of contacting is carried out at a pressure ranging between 1 kg/ cm² and 10 kg/ cm².

9. The process as claimed in claim 1, wherein said step of contacting is carried out at a gas hourly space velocity (GHSV) ranging between 10,000 per hour and 20,000 per hour.

10. The process as claimed in claim 1, wherein the shape of said macroporous alpha alumina is selected from the group of shapes consisting of bead shape and disc shape.

## Patentansprüche

1. Verfahren zum Entfernen von Metallverunreinigungen aus einem verunreinigten Flüssigkeitsstrom, den Schritt umfassend, den Flüssigkeitsstrom mit makroporösem Alpha-Aluminiumoxid in Kontakt zu bringen, um einen Flüssigkeitsstrom zu erhalten, der weniger als 50 ppm Metall enthält, wobei der Flüssigkeitsstrom ein Strom von Mineralsäure(n) ist.

2. Verfahren nach Anspruch 1, wobei die Metallverunreinigung mindestens ein elementares Metall oder Metalloxid ist.

3. Verfahren nach Anspruch 1, ferner einen Schritt des Gewinnens des verbrauchten makroporösen Alpha-Aluminiumoxids und Regenerierens des aktiven makroporösen Alpha-Aluminiumoxids daraus umfassend, indem das verbrauchte makroporöse Alpha-Aluminiumoxid mit mindestens einem gereinigten Strom von Mineralsäure(n) gespült wird, wobei die Mineralsäure mindestens eine ätzende Säure ist, die aus der Gruppe bestehend aus Salzsäure, Fluorwasserstoffsäure, Bromwasserstoffsäure, Salpetersäure, Phosphorsäure, Schwefelsäure, Borsäure und Perchlorsäure gewählt wird.

4. Verfahren nach Anspruch 1, wobei der Flüssigkeitsstrom ein Salzsäurestrom ist.

5. Verfahren nach Anspruch 1, wobei die Metallverunreinigung mindestens ein Metall aus der Gruppe bestehend aus Eisen, Nickel, Kupfer, Chrom, Blei, Zink, Mangan und Oxide derselben ist.

6. Verfahren nach Anspruch 1, wobei die Metallverunreinigung Eisenoxid ist.

7. Verfahren nach Anspruch 1, wobei der Schritt des In-Kontakt-Bringens bei einer Temperatur im Bereich zwischen 20 °C und 200 °C ausgeführt wird.

8. Verfahren nach Anspruch 1, wobei der Schritt des In-Kontakt-Bringens bei einem Druck im Bereich zwischen 1 kg/cm² und 10 kg/cm² ausgeführt wird.

9. Verfahren nach Anspruch 1, wobei der Schritt des In-Kontakt-Bringens bei einer stündlichen Raumgeschwindigkeit des Gases (GHSV) im Bereich zwischen 10.000 pro Stunde und 20.000 pro Stunde ausgeführt wird.

10. Verfahren nach Anspruch 1, wobei die Form des makroporösen Alpha-Aluminiumoxids aus der Gruppe bestehend aus perlenförmig und scheibenförmig gewählt wird.

## Revendications

1. Procédé d'élimination des contaminants métalliques d'un courant de fluide contaminé, comprenant l'étape consistant à mettre en contact ledit courant de fluide avec de l'alumine alpha macro poreuse dans le but d'obtenir un courant de fluide contenant moins de 50 ppm de métal, dans lequel ledit courant de fluide est un courant d'acide(s) minéral(aux).

2. Procédé selon la revendication 1, dans lequel ledit contaminant métallique est au moins l'un des métaux élémentaires et des oxydes métalliques.

3. Procédé selon la revendication 1, comprenant en outre une étape consistant à obtenir de l'alumine alpha macro poreuse usée et à en régénérer de l'alumine alpha macro poreuse active par lavage de ladite alumine alpha macro poreuse usée avec au moins un courant purifié d'acide(s) minéral(aux), dans lequel ledit acide minéral est au moins un acide corrosif choisi dans le groupe constitué par l'acide chlorhydrique, l'acide fluorhydrique, l'acide bromhydrique, l'acide nitrique, l'acide phosphorique, l'acide sulfurique, l'acide borique et l'acide perchlorique.

4. Procédé selon la revendication 1, dans lequel ledit courant de fluide est un courant d'acide chlorhydrique.

5. Procédé selon la revendication 1, dans lequel ledit contaminant métallique est au moins un métal du groupe constitué par le fer, le nickel, le cuivre, le chrome, le plomb, le zinc, le manganèse et leurs oxydes.

6. Procédé selon la revendication 1, dans lequel ledit contaminant métallique est de l'oxyde de fer.

7. Procédé selon la revendication 1, dans lequel l'étape de mise en contact est réalisée à une température comprise entre 20°C et 200°C.

8. Procédé selon la revendication 1, dans lequel l'étape de mise en contact est réalisée à une pression comprise entre 1 kg/cm² et 10 kg/cm².

9. Procédé selon la revendication 1, dans lequel l'étape de mise en contact est réalisée à une vitesse spatiale horaire des gaz (VSHG) comprise entre 10 000 par heure et 20 000 par heure.

10. Procédé selon la revendication 1, dans lequel la forme de ladite alumine alpha macro poreuse est choisie dans le groupe de formes consistant en une forme de perle et une forme de disque.
